# EUROPEAN PATENT APPLICATION

(11) **EP 4 656 187 A1**
(43) Date of publication of application: **03.12.2025**
(21) Application number: 24178973.4
(22) Date of filing: 30.05.2024
(51) Int. Cl.: A61K 9/50

(54) **METHOD FOR THE PRODUCTION OF EXTRACELLULAR VESICLES LOADED WITH A MOLECULE OF INTEREST AND COMPOSITION COMPRISING SUCH EXTRACELLULAR VESICLES**

(71) Applicant: ConvEyXo, 6041 Charleroi (BE)
(72) Inventor: VAN MECHELEN, Marcelle, 6041 Charleroi (BE); HUBAUX, Roland, 5030 Gembloux (BE); PRIEELS, Jean Paul, 1380 Lasne (BE)
(74) Representative: Calysta NV

(57) **Abstract**

Method for the production of extracellular vesicles, EVs, loaded with a molecule of interest in a bioreactor containing a porous three-dimensional, 3D, scaffold, wherein the method comprises a seeding of the scaffold with a starting number of adherent immortalized cells resuspended after detachment, an attachment of the cells on said scaffold, an expansion of the cells on said scaffold up to a second number of adherent cells, defining a multiplication factor of at least 5 for a single expansion step, a production of EVs by said cells, a phase of loading said EVs with said molecule of interest, a harvest of EVs.

## Description

### Technical Field

The present invention relates to a method for the production of extracellular vesicles loaded with a molecule of interest compatible with the pharmaceutical industry, for example exosomes loaded with a molecule of interest.

The present invention also relates to a pharmaceutical composition containing extracellular vesicles loaded with a molecule of interest obtained by the method according to the present invention.

The present invention also pertains to a pharmaceutical composition containing extracellular vesicles loaded with a molecule of interest.

### State of the art

Exosomes are extracellular vesicles with a diameter between 30 and 150 nm and are secreted by a multitude of cell types. Exosomes are involved in several cellular processes such as intercellular communication, antigen presentation, transfer of proteins, messenger RNAs, siRNAs, and/or microRNAs from one cell to another. The function of exosomes is altered in many diseases such as cancers, which suggests their importance as a diagnostic tool and/or in therapeutic applications, for example in acellular therapies as a vehicle to deliver therapeutic molecules, in immunotherapy, to treat different types of cancer and/or inflammatory and cardiovascular neurodegenerative diseases, diabetes, etc.

Some protocols for isolating and purifying exosomes and extracellular vesicles are known, and exosomes have already been successfully used in cell-free therapy studies.

However, although some exosome isolation and purification protocols exist, it is important to develop exosome production and purification methods that allow for batch-to-batch consistent pharmaceutical-grade exosome production at large scale, with high yield, while being fast and inexpensive so as to increase the use of exosomes as a cell-free therapy tool in medical practice, for the benefit of the greatest number of patients.

The production of exosomes must meet several constraints: it is necessary to obtain a culture of cells producing high density exosomes of high quality, such as for example mesenchymal stem cells, and to put these cells under adequate conditions to maximize their production of exosomes, in such a way as to obtain a quantity of exosomes sufficient to be able to be used in therapeutic applications. Extracellular vesicles from mesenchymal stem cells have been shown to offer broad regenerative potential in many diseases.

There are methods for producing exosomes using adherent cell culture on a porous three-dimensional scaffold. The porous three-dimensional scaffold being known to ensure a high cell density of cells in culture. However, these methods either use complex devices that can cause, among other things, premature wear of the support, or are not easily automatable for repeated and reproducible productions of exosomes necessary for current medical practice.

In addition, exosomes can also be loaded with small-molecules and can act as a therapeutic cargo delivery system. Different strategies already exist for the loading of exosomes with small-molecules allowing a targeted delivery of drug molecules by exosomes (Zeng H. et al. 2023, Current strategies for exosome cargo loading and targeting delivery. Cells 12(10): 1416, DOI: 10.3390/cells12101416).

In that context, EP3706796 describes extracellular vesicle (EV) therapeutics, wherein the EVs comprise endogenously loaded RNA therapeutics such as mRNAs, circular RNAs, miRNAs, shRNAs and various other RNA therapeutics agents. However, even if this document and the prior art extol the merits of their technology, currently, no efficient production and loading strategies allow reaching a sufficient amount of loaded exosomes with sufficient loading of molecules of interest (therapeutic molecules) to be used as a therapeutic means in daily medical practice while being reliable, sufficiently generalizable and reproducible and remaining affordable in terms of production costs.

Consequently, there is a need to improve the current production and loading methods by making them more efficient, industrially applicable both in terms of the quantity of loaded exosomes produced and in terms of production costs, and this with a production of loaded exosomes with constant pharmaceutical quality regardless of the production batch.

### Objectives of the invention

The present invention aims to overcome the drawbacks of the state of the art, in particular those described above.

In particular, the present invention proposes to provide a method for producing loaded exosomes with a molecule of interest making it possible to reach an industrial scale of production which is profitable by minimizing production costs.

Another objective of the present invention is to provide a method of producing loaded extracellular vesicles of constant pharmaceutical quality, regardless of the production batch, which meets GMP standards.

The present invention also aims to provide a pharmaceutical composition containing extracellular vesicles loaded with a molecule of interest having high and efficient anti-inflammatory properties able to be used as a therapeutic option in daily medical practices for chronic inflammatory pathology and/or an age-related pathology.

### Summary of the invention

To achieve the aforementioned objectives, the present invention provides a method for the production of extracellular vesicles loaded with one or several molecule of interest compatible with the pharmaceutical industry, for example exosomes loaded with one or several molecule of interest comprising the following steps:
- a supply of a bioreactor chamber containing a porous three-dimensional scaffold by a cell culture medium via a supply port, and, after a predefined duration, an exit via an outlet port,
- seeding said porous three-dimensional scaffold with a starting number of cells (starting number of adherent cells resuspended after detachment) in a volume of culture medium,
- a phase of attachment of said cells to form adherent cells on said porous three-dimensional scaffold,
- an expansion of the adherent cells on said porous three-dimensional scaffold up to a second number of adherent cells, said second number of adherent cells divided by said starting number defining a multiplication factor of at least 5 for a single expansion step in the said scaffold,
- a production of extracellular vesicles by said adherent cells during a production phase, subsequent to or concomitantly with the expansion, during which the vesicles are produced and excreted in the culture medium to give a culture medium enriched with extracellular vesicles,
- preferably, a first purification of the culture medium enriched with extracellular vesicles in order to produce a purified culture medium enriched with extracellular vesicles,- a loading of said extracellular vesicles with said one or several molecule of interest, subsequently to or concomitantly with the production,
- preferably, a (second) purification arranged to remove unloaded extracellular vesicles in order to produce a purified culture medium enriched with extracellular vesicles loaded with a molecule of interest,
- a harvest of extracellular vesicles.

As it can be seen, according to the present invention, a bioreactor with a porous three-dimensional scaffold (fixed bed) is seeded with adherent cells.

According to the present invention, the adherent cells resuspended after detachment in a volume of culture medium are preferably obtained by detachment, for example by trypsinisation, of the adherent cells from a culture of adherent cells and resuspension of said adherent cells in the volume of culture medium.

After or concomitantly to the expansion (growth phase), extracellular vesicles production phase is started, for example by stress or chemical induction, and the vesicles are produced and excreted in the culture medium to give a culture medium enriched with extracellular vesicles. After or concomitantly to the production phase, the extracellular vesicles can be loaded with at least one molecule of interest leading to a high production of extracellular vesicles of high and constant quality loaded with molecules of interest.

Surprisingly, while the density of cells after the growing phase is very high, it was found that high extracellular vesicle (EV) concentration is achieved with EV of high quality in the presence of few death cells in the harvesting solution of the extracellular vesicles. EV of high quality in the presence of few death cells in the harvesting solution will also facilitate the loading of EV with molecules of interest when the loading occurs after the harvest phase and subsequent purification. By the unique combination of this adequate environment with a culture of an adherent cell line, which are, advantageously, very stable between each generation, and with, preferably, a multiplication factor of at least 5, allows not only to reach but also to maintain a very high yield of cells of high and constant quality with few death cells. This cell culture of high yield and constant quality regardless of the production batch promotes a production of loaded extracellular vesicles of high and constant pharmaceutical quality, which meets GMP standards while reaching an industrial scale of production which is profitable by minimizing production costs of a cycle of production.

Other embodiments of the method for the production of extracellular vesicles loaded with a molecule of interest according to the present invention are indicated in the appended claims.

The present invention also relates to a pharmaceutical composition containing extracellular vesicles loaded with a molecule of interest obtained by the method according to the present invention.

Other embodiments of the pharmaceutical composition of the present invention obtained by the method according to the present invention are indicated in the appended claims.

The present invention also relates to a pharmaceutical composition comprising a therapeutically effective amount of extracellular vesicles loaded with a molecule of interest wherein said molecule of interest comprises at least a circular RNA, and wherein said extracellular vesicles are extracellular vesicles obtained from adherent immortalized mesenchymal stem cell.

Alternatively, the present invention also relates to a pharmaceutical composition comprising a therapeutically effective amount of extracellular vesicles loaded with a molecule of interest wherein said molecule of interest comprises at least a miRNA, and wherein said extracellular vesicles are extracellular vesicles obtained from adherent immortalized mesenchymal stem cell.

Alternatively, the present invention also relates to a pharmaceutical composition comprising a therapeutically effective amount of extracellular vesicles loaded with a molecule of interest wherein said molecule of interest comprises at least a circular RNA and at least a miRNA.

According to the present invention, the term "a therapeutically effective amount" preferably means an amount able to generate even transiently a modification in the status of a disease and/or a pathology, for example by modifying even transiently the expression levels of nucleic acids and/or proteins overexpressed and/or downregulated in the disease and/or in the pathology.

Indeed, it was shown that extracellular vesicles (EVs) obtained from adherent immortalized mesenchymal stem cell, wherein the extracellular vesicles are loaded with at least a circular RNA and/or at least a miRNA is particularly efficient to improve the anti-inflammatory properties of the adherent immortalized stem cell-derived extracellular vesicles, making of the loaded EVs a very useful therapeutic tool.

Other embodiments of the pharmaceutical composition according to the present invention are indicated in the appended claims.

The present invention also relates to a pharmaceutical composition according to the present invention, for use in the treatment of a chronic inflammatory pathology and/or an age-related pathology, wherein the chronic inflammatory pathology and/or the age-related pathology is selected from a group of chronic inflammatory pathology and/or age-related pathology consisting of osteoarthritis, Alzheimer disease, liver disease, diabetic diseases, such as More preferably, type 2 diabetic conditions, lung fibrosis, atherosclerosis, facet joint osteoarthritis, multiple sclerosis, inflammation, but also cancer.

Other embodiments of the pharmaceutical composition according to the present invention for use in the treatment of a chronic inflammatory pathology and/or an age-related pathology are indicated in the appended claims.

### Detailed description of the invention :

Other features and advantages of the present invention will be derived from the following non-limiting description, and with reference to the following figure:
According to the present invention, preferably, the term "extracellular vesicle" (EV) is understood to mean lipid bilayer-delimited particles that can be secreted by numerous cell types. The size of the extracellular vesicles ranges from 20 nm to more than 1000 nm, wherein exosomes represent extracellular vesicles with a size range between 30 and 150 nm, while microvesicles may have a size ranging from 100 nm to 1000 nm and apoptotic bodies have a size greater than 1000 nm.

According to the present invention, the term "immortalized cells" preferably means very stable and with a very large capacity of proliferation, the latter preferably meaning cells able to have a generation number superior to 50, preferably superior to 70, more preferably superior to 100. According to the present invention, the term "generation number" preferably means a number of a successive series of cell subcultures that can be obtained from an initial cell culture without modifying, or without modifying substantially, the cellular properties of the initial cell culture.

According to the present invention, preferably, the term "stress or chemical induction" that may allow the starting of the extracellular vesicles production phase comprise (i) a modulation of the culture conditions such as for example modifying the temperature of culture, modifying the pH of the culture medium, modifying the level of 02, (ii) a modification of the biological conditions through modifying the amount of cytokines and/or other growth factors and/or other cell culture medium supplements and/or other components of the cell culture media formulation (iii) a chemical stimulation such as for example the addition of sulfhydryl blocking agents, agents for calcium influx, H₂O₂, and/or vesiculation compound, and/or specific inhibitors or activators of cell signalling, or any compound involved in vesicle production, trafficking, and excretion, (iv) physical stimulation such as for example shearing stress, high-frequency ultrasound, irradiation, and/or (v) cell membrane disruption.

According to the present invention, preferably, the (printed and/or3D) scaffold is designed through a computer-aided design, CAD. Preferably, the volumes and/or the specific surface area of the scaffold are determined by high resolution X-ray computed tomography.

According to the present invention, preferably, the term "scaffold" is understood to mean a fixed bed.

According to the present invention, preferably, the term "a loading of said extracellular vesicles with said molecule of interest" means an intravesicular loading of said molecule of interest into said extracellular vesicles. The loading of said extracellular vesicles with said molecule of interest can be performed intracellularly, when the vesicles are still inside the cells and are not yet excreted, and/or can be performed extracellularly, after the excretion of the vesicles outside of the cells.

Preferably, the bioreactor chamber is included in a bioreactor (bioreactor system) comprising, in addition of the bioreactor chamber containing a porous three-dimensional scaffold, a medium recirculation loop comprising, preferably, at least one medium reservoir with additional in- and outlets.

Preferably, the bioreactor system comprises one or multiple solutions for on-line and/or in-line process monitoring and/or control including, but not limited to, in-line probes and/or sensors, mixing devices for fluids and/or gasses and feed and waste lines.

Preferably, the porous three-dimensional scaffold is a porous three-dimensional printed scaffold. More preferably, the porous three-dimensional scaffold having a specific surface area and a total volume in a ratio comprised between 25 m²/m³ and 250 m²/m³, more preferably between 45 m²/m³ and 250 m²/m³, even more preferably between 50 m²/m³ and 250 m²/m³, advantageously between 60 m²/m³ and 250 m²/m³. Indeed, a porous three-dimensional printed scaffold (fixed bed), by contrast to chemically obtained 3D scaffold and/or by contrast to 2D scaffold stacked or stuffed to make 3D scaffold, allows to control the scaffold geometry in such a way that there is no preferred fluid pathway inside the scaffold. The porous three-dimensional scaffold with said controlled geometry as a result of the 3D printing method of producing the scaffold has a specific surface area and a total volume. The controlled geometry of the scaffold in 3 dimensions, instead of random geometries of stacked 2D geometries, enables a 3 dimensional fluid flow, thus avoiding preferential pathways.

According to the present invention, preferably, the porous three-dimensional scaffold is composed of one 3D motif or a combination of several 3D motifs. According to the present invention, preferably, the total volume of the porous three-dimensional scaffold is the volume of the 3D motifs or of all 3D motifs composing the scaffold.

The scaffold 3D has advantageously an optimized geometry and/or, preferably, the 3D printing method offers an environment where the cells can grow in each direction giving rise to an homogeneous cell distribution, in an uniform way, regardless of the position of the cells within the scaffold due to the absence of preferential pathway (homogeneous flow path) while remaining in close contact with their secreted metabolites promoting the growth of the cells. Moreover, the 3D printed scaffold with a controlled geometry having a specific surface area to volume ratio comprised between 25 m²/m³ and 250 m²/m³ offers not only an adequate environment to promote but also to maintain an uniform cell culture. Preferably, the molecule of interest is shRNA, miRNA, mRNA, gRNA, pri-miRNA, pre-miRNA, circular RNA, piRNA, tRNA, rRNA, snRNA, IncRNA (long non-coding RNA), ribozymes, mini-circle DNA, plasmid DNA, circular RNA, siRNA, agomir, antagomir or a combination thereof. More preferably, the molecule of interest is circular RNA and/or miRNA.

In the context of the present invention, by IncRNA (long non-coding RNA), it is preferably meant a non-coding RNA transcript with a nucleotide sequence equal or longer than 200 nucleotides.

In the context of the present invention, by "miRNA", it is preferably meant a small, single-stranded, non-coding RNA molecules containing a nucleotide sequence length of less than 100 nucleotides, more preferably less than 50 nucleotides, even more preferably less than 30 nucleotides. Advantageously, miRNAs can act by destabilizing messenger RNAs.

In the context of the present invention, by "circular RNA", it is preferably meant a type of single-stranded RNA, which forms a covalently closed continuous loop, wherein the 3' and 5' ends present in an RNA molecule have been joined together.

In the context of the present invention, by "agomir", it is preferably meant a chemically-modified double-strand miRNA mimic with modified antisense strand, wherein agomirs are able to act as a mature endogenous miRNA.

In the context of the present invention, by "antagomir", it is preferably meant a chemically-modified single-strand miRNA inhibitor.

Preferably, the adherent cells are adherent immortalized cells. Preferably, the adherent cells are cells forming part of a group of cells consisting of immortalized immune regulatory cells, immortalized mesenchymal stem cells, immortalized Wharton's Jelly derived-mesenchymal stem cells, immortalized bone marrow-derived mesenchymal stromal cells, immortalized hTERT-mesenchymal stem cells, and/or immortalized induced pluripotent stem cells or cells derived from induced pluripotent stem cells, preferably immortalized mesenchymal stem cells.

Preferably, the adherent cells are clonal cells.

Preferably, the adherent immortalized cells are engineered to stably express said molecule of interest, when the said molecule of interest is unmodified RNA or DNA.

Preferably, the loading the extracellular vesicles with the molecule of interest is performed through
(i) a transfection step of the adherent cells during or after said expansion with a vector encoding said molecules of interest and/or directly with the molecules of interest, and/or
(ii) a mechanoporation step after the harvest of extracellular vesicles, and/or
(iii) the intracellular incorporation of the molecule of interest stably expressed by the engineered adherent immortalized cells into the extracellular vesicles.

Preferably, the mechanoporation step is performed after the transfection step and/or the intracellular incorporation of the molecule of interest stably expressed by the engineered adherent immortalized cells into the extracellular vesicles.

In the context of the present invention, by "a mechanoporation step" it is preferably meant a passage of the extracellular vesicles that have been harvest through a nanochannel, wherein the dimension of the nanochannel, for example the diameter and/or the width of the nanochannel is such that the membrane of the extracellular vesicles is mechanically squeezed by the passage through the nanochannel leading to the formation of holes in the membrane of the extracellular vesicles allowing the molecule of interest to enter into the extracellular vesicles.

Preferably, the mechanoporation step is performed by the passage of a solution containing the extracellular vesicles that have been harvested through a micro-nano fluidic chip, wherein the micro-nano fluidic chip is a chip comprising a combination of micro-channels and nano-channels arranged to allow an inlet of a solution comprising extracellular vesicles through a microchannel and to squeeze the extracellular vesicles by their passage through a nanochannel Preferably the micro-nano fluidic chip comprises
- at least one inlet for the entry of the solution,
- at least one outlet for the exit of the solution containing the squeezed extracellular vesicles,
- at least one nano-channel array layer and at least one micro-channel array layer,
wherein the nano-channel array layer and the micro-channel array layer are alternately stacked. Preferably, the solution also contains the molecule of interest to be loaded into the extracellular vesicles.

Preferably, the method according to the present invention further comprises, after the production step, a first purification step of the culture medium enriched with extracellular vesicles in order to produce a purified culture medium enriched with extracellular vesicles. Indeed, the first purification step before the loading step permits avoiding that contaminants, such as for example proteins and undesired nucleic acids, present in the culture medium being loaded together with the molecule of interest into the extracellular vesicles. More preferably, the first purification comprises :
- a clarification step with one or several purification steps wherein the one or several purification steps having a retention rate comprised between 5 µm and 0.1 µm, more preferably between 2 µm and 0.15 µm, the clarification step being arranged to clear intact cells, cell debris and aggregates,
- one or several tangential flow filtration arranged to concentrate the culture medium enriched with extracellular vesicles,
- optionally, an enzymatic treatment arranged to digest residual nucleic acids,
- an anion exchange chromatography arranged to remove residual DNA and/or proteins.

Preferably, before the harvest of extracellular vesicles and after the first purification step, the method according to the present invention further comprises, a second purification of the extracellular vesicles wherein the second purification is arranged to remove free nucleic acids in order to produce a purified culture medium enriched with extracellular vesicles. More preferably, the second purification comprises :
- an anion exchange chromatography arranged to remove residual DNA and/or proteins,
- a tangential flow filtration arranged to equilibrate the exosomes solution in a saline solution wherein the saline solution having a physiological pH comprised between 7.2 and 7.6, preferably 7.4.

Preferably, all the steps of the method according to the present invention are performed under good manufacturing practices, GMP, conditions.

Preferably, in the pharmaceutical composition containing extracellular vesicles loaded with a molecule of interest obtained by the method according to the present invention, the extracellular vesicles are adherent immortalized cell-derived exosomes. More preferably, the extracellular vesicles are immortalized mesenchymal stem cell-derived exosomes.

Preferably, the pharmaceutical composition containing extracellular vesicles loaded with a molecule of interest is under a liquid composition or a frozen composition or a lyophilized composition or a spray dried composition or a gel composition.

Preferably, the extracellular vesicles are conditioned into a pharmaceutically acceptable liquid buffer.

Preferably, the extracellular vesicles are exosomes.

Preferably, in the pharmaceutical composition containing extracellular vesicles loaded with a molecule of interest obtained by the method according to the present invention, the molecule of interest comprises at least one circular RNA. More preferably, the molecule of interest comprises at least one circular RNA, wherein the at least one circular RNA represents at least 10%, more preferably 20%, 30%, 40%, 50%, 60% of all the circular RNA present in the extracellular vesicles (copy number of the at least one circular RNA / total copy number of circular RNA in the extracellular vesicles). Preferably, the at least one circular RNA possess at least 2, more preferably 3, 4 binding sites for its target miRNA(s). More preferably, the at least one circular RNA is able to regulate, preferably down-regulate, one or several miRNAs wherein said one or several miRNAs is upregulated or overexpressed in a particular pathology or disease as compared to its expression level in the corresponding normal cell or tissue, such as for example a chronic inflammatory pathology and/or an age-related pathology.

Alternatively, or in addition, the molecule of interest comprises at least one miRNA. More preferably, the molecule of interest comprises at least one miRNA, wherein the at least one miRNA represents at least 10%, more preferably 20%, 30%, 40%, 50%, 60% of all miRNA present in the extracellular vesicles (copy number of the at least one miRNA / total copy number of miRNA in the extracellular vesicles). More preferably, the at least one miRNA is downregulated and/or under-expressed in a particular pathology or disease, or in the patient affected by a disease, such as for example a chronic inflammatory pathology and/or an age-related pathology. More preferably, the molecule of interest comprises a plurality of miRNAs. Indeed, the presence of a plurality of miRNAs loaded into extracellular vesicles will improve the therapeutic responses of the pharmaceutical composition.

Preferably, the pharmaceutical composition containing extracellular vesicles loaded with a molecule of interest obtained by the method according to the present invention is for use in the treatment of a chronic inflammatory pathology and/or an age-related pathology.

Preferably, the molecule of interest comprises at least a circular RNA and at least a miRNA, more preferably wherein the at least one circular RNA is able to regulate, preferably down-regulate, one or several miRNAs wherein said one or several miRNAs is upregulated or overexpressed in a particular pathology or disease, and wherein the at least one miRNA is downregulated and/or under-expressed in such particular pathology or disease.

Preferably, the molecule of interest comprises a combination of miR-146 and anti-miR-155.

Alternatively, the molecule of interest comprises a combination of miR-140 and miR-146.

Alternatively, the molecule of interest comprises a combination of miR-140 and anti-miR-155.

Alternatively, the molecule of interest is a circular RNA (circRNA).

Alternatively, the molecule of interest comprises (i) miR-146 or miR-140, and (ii) a circular RNA (circRNA).

Alternatively, the molecule of interest comprises (i) miR-140 and miR-146, and (ii) a circular RNA (circRNA).

Alternatively, the molecule of interest comprises (i) miR-146 and miR-140, and/or (ii) hsa-circRNA_0001236.

More preferably, the molecule of interest comprises:
(i) at least one circular RNA, wherein the at least one circular RNA represents at least 10%, more preferably 20%, 30%, 40%, 50%, 60% of all the circular RNA present in the extracellular vesicles (copy number of the at least one circular RNA / total copy number of circular RNA in the extracellular vesicles), and
(ii) at least one miRNA, wherein the at least one miRNA represents at least 10%, more preferably 20%, 30%, 40%, 50%, 60% of all miRNA present in the extracellular vesicles (copy number of the at least one miRNA / total copy number of miRNA in the extracellular vesicles).

Preferably, in the pharmaceutical composition containing extracellular vesicles loaded with a molecule of interest according to the present invention, the extracellular vesicles are exosomes obtained from adherent immortalized mesenchymal stem cell.

Preferably, in the pharmaceutical composition containing extracellular vesicles loaded with a molecule of interest for use in the treatment of a chronic inflammatory pathology and/or an age-related pathology according to the present invention, the molecule of interest comprises at least a circular RNA and at least a miRNA.

Preferably, in the pharmaceutical composition containing extracellular vesicles loaded with a molecule of interest for use in the treatment of a chronic inflammatory pathology and/or an age-related pathology, more preferably for use in the treatment of a chronic inflammatory pathology and/or an age-related pathology in humans according to the present invention, the molecule of interest comprises
- at least a circular RNA chosen in the group of circular RNA consisting of hsa-circRNA_0001236, hsa-circRNA_0025554, hsa-circRNA_0035855, hsa-circRNA_0037422, hsa-circRNA_0091178, hsa-circRNA_0012265, hsa-circRNA_0039053, hsa-circRNA_0002643, hsa-circRNA_0082734, hsa-circRNA_0076177, circ-BRWD1, circ-001846, hsa-circRNA_0068087, circ_PUM1, circ_0138959, circ_PPP1 CC, circ_PPM1F, circ_ZNF532, has-circ_0105015, circ_NFIC, circ_0039411, circ_OAS3, ciRS-7, circ_ARF3, hsa-circ_0000515, circ_TUBD1, circ_Ttc3, circ_TLK1, circ_VMA21, circ_0068888, circ_GRN, circ_0003204, circ_AFF2, circ_0008360, circ_FBXW7, circ_0128846, circ_0134111, hsa-circ_0005567, circ_ANKRD36, circ_SEC24A, circ_HIPK3, circ_0001105, has-circ_0003353, circ_ZNF652, circ_HIPK3, circ_0026579, circ_VMA21, circ_406961, circ_TXNRD1, circ_Bbs9, circ_RSF1, circ_CDKN2B-AS1, circ_0093884, circ_0000950, circ_HECTD1, circ_DLGAP4, circRNA-2960, circ_Plek, circ_014301, circ_Prkcsh, circ_SMEK1, circ_0004354, circ_0040039, circ_PPP1 CC, circ_PPM1F, circ_OAS3, circ_ARF3, circANRIL, circRNA_103017, circRNA_101 128, and circRNA_059914, mcircRasGEF1B, and
- at least a miRNA chosen in the group of miRNA consisting of hsa-miR-155 (NR_030784), hsa-miR-146a (NR_029701), hsa-miR-140 (NR_029681), hsa-miR-124 (NR_029668, NR_029670), hsa-miR-689, hsa-miR-126 (NR_029695), hsa-miR-132 (NR_029674), hsa-miR-122 (NR_029667), hsa-miR-10a (AJ550394), hsa-miR-7, hsa-miR-125b (NR_029671), hsa-miR-31 (NR_029505), hsa-miR-210 (AJ550413), hsa-miR-24 (NR_029496.1, NR_029497), hsa-miR-149 (NR_029702), hsa-miR-181 (AY194157), hsa-miR-150 (NR_029703), hsa-miR-143 (AJ535834), hsa-miR-9 (NR_029691, NR_029692), hsa-miR-142 (NR_029683), hsa-miR-223 (AJ550427), hsa-miR-21 (NR_029493), hsa-miR-132 (NR_029674), hsa-miR-92a (NR_029509, NR_029508), hsa-miR-200 (NR_029834, NR_029639,NR_029779), hsa-miR-23a (NR_029495), hsa-miR-27a (NR_029501), hsa-miR29c (NR_029832), hsa-miR-138 (NR_029700, NR_029680), hsa-miR-34a (NR_029610), hsa-miR-34c (NR_029840), hsa-Let-7a (NR_029478, NR_029477, NR_029476), hsa-miR-1246 (NR_031648), hsa-miR-449a (NR_029960), hsa-miR-1277 (NR_031685), hsa-miR-214 (AJ550417), hsa-miR-27b (NR_029665), hsa-miR-199a (NR_029618, NR_029586), hsa-miR-183 (NR_029615), hsa-miR-118 (AF480516).

Preferably, the pharmaceutical composition according to the present invention is characterized in that said pharmaceutical composition is administered intraarticular, Intra nasal, as a spray, topically.

More preferably, the liver disease comprises Hepatic steatosis, Liver fibrosis, NAFLD (Nonalcoholic fatty liver disease), Radiation-induced liver disease, Radiation-induced liver fibrosis,

More preferably, the inflammation comprises Nervous inflammatory diseases, immune disorders, Inflammatory bowel disease, Crohn's disease, Ulcerative Colitis, Psoriasis, Asthma, Psoriatic Arthritis, Rheumatoid Arthritis, Psoriasis, Lupus, Chronic Obstructive Pulmonary Disease.

Preferably, in the composition containing extracellular vesicles loaded with a molecule of interest for use in the treatment of Alzheimer's disease, the molecule of interest is at least one miRNA and at least one circular RNA chosen in the group of circular RNA consisting of hsa_circ_0001946 (27929395), hsa_circ_0003391 (33424579), hsa_circ_0030777 (32315771), hsa_circ_0031258 (32315771), hsa_circ_0032253 (32315771), hsa_circ_0000775 (32315771), hsa_circ_0047285 (32315771), hsa_circ_0002945 (32315771), hsa_circ_0007556 (33003364), hsa_circ_0131235 (33704916), hsa_circ_0003594 (30887246), hsa_circ_0003611 (32315771), hsa_circRNA_000843 (31834549), hsa_circRNA_101618 (31834549), hsa_circRNA_103366 (31834549), hsa_circRNA_103936 (31834549), hsa_circRNA_104395 (31834549), hsa_circRNA_402904 (31834549), hsa_circRNA_403472 (31834549), hsa_circRNA_405619 (31834549).

Alternatively, in the composition containing extracellular vesicles loaded with a molecule of interest for use in the treatment of Alzheimer disease, the molecule of interest comprises (i) at least one miRNA chosen in the group consisting of miR-146a and miR-23a, and (ii) at least an anti-mir-155. More preferably, the molecule of interest comprises (i) at least one miRNA chosen in the group of miRNAs consisting of miR-146a, miR-140, miR-126, miR-132, and (ii) at least an anti-mir-155 in an amount equal or superior to 10%, more preferably 20%, 30%, 40%, 50%, 60% of all miRNA sequences present in the extracellular vesicles (copy number of at least one miRNA chosen in the group consisting of miR-146a and miR-23a and an anti-mir-155 present in the extracellular vesicles towards the total copy number of all miRNA sequences present in the extracellular vesicles). Even more preferably, in the composition containing extracellular vesicles loaded with a molecule of interest for use in the treatment of Alzheimer disease, the molecule of interest consists of a combination of (i) at least one miRNA chosen in the group of miRNAs consisting of miR-146a, miR-23a, and (ii) anti-miR-155.

Preferably, in the composition containing extracellular vesicles loaded with a molecule of interest for use in the treatment of Atherosclerosis, the molecule of interest is at least one miRNA and at least one circular RNA chosen in the group of circular RNA consisting of hsa_circ_0010283 (32945389), hsa_circ_0000231 (33439448), hsa_circ_0093887 (33844344), hsa_circ_0003573 (28946214), hsa_circ_0000345 (28946214), hsa_circ_0029589 (32271446), hsa_circ_0007478 (33526034), hsa_circ_0003645 (32554046), hsa_circ_0044073 (30864721), hsa_circ_0003204 (33249762), hsa_circ_0004872 (33592319), hsa_circ_0004264 (28946214), hsa_circ_0006896 (33649864), hsa_circ_0008574 (27539542), hsa_circ_0003575 (28946214), hsa_circ_0001946 (32053286). According to the present invention, the above numbers into brackets are the PMID number of the articles referring to the circular RNAs.

Preferably, in the composition containing extracellular vesicles loaded with a molecule of interest for use in the treatment of facet joint osteoarthritis, the molecule of interest is at least one miRNA and at least one circular RNA chosen in the group of circular RNA consisting of hsa_circ_0005127 (29470979), hsa_circ_0029067 (29470979), hsa_circ_0053743 (29470979), hsa_circ_0063781 (29470979), hsa_circ_0071896 (29470979), hsa_circ_0072668 (29470979), hsa_circ_0087537 (29470979). According to the present invention, the above numbers into brackets are the PMID number of the articles referring to the circular RNAs.

Preferably, in the composition containing extracellular vesicles loaded with a molecule of interest for use in the treatment of hepatic steatosis, the molecule of interest is at least one miRNA and at least one circular RNA chosen in the group of circular RNA consisting of hsa_circRNA_014724 (28717649), hsa_circ_014724 (28717649), hsa_circRNA_004183 (28717649), hsa_circ_002082/hsa_circ_0000271 (28717649), hsa_circ_0004183, hsa_circRNA_000367 (28717649), hsa_circRNA_002082 (28717649), hsa_circ_000367 (29218114), hsa_circ_0046367 (29018509), hsa_circ_0046366 (29391755), hsa_circRNA_004121 (28717649), hsa_circ_004121 (28717649), hsa_circRNA_007850 (28717649), hsa_circ_0007850. According to the present invention, the above numbers into brackets are the PMID number of the articles referring to the circular RNAs.

Preferably, in the composition containing extracellular vesicles loaded with a molecule of interest for use in the treatment of multiple sclerosis, the molecule of interest is at least one miRNA and at least one circular RNA chosen in the group of circular RNA consisting of hsa_circ_0024892 (28651352), hsa_circ_0000517 (28651352), hsa_circ_0000519 (28651352), hsa_circ_0000520/hsa_circ_001846 (28651352), hsa_circ_0005402/hsa_circ_0000518/hsa_circ_000167 (28651352), hsa_circ_0035560/hsa_circ_0005402/hsa_circRNA_101539/hsa_circRNA_101541 (28651352), hsa_circ_0106803 (28272342), hsa_circ_0043813 (30619471), hsa_circRNA_024892 (29197342), hsa_circ_0001400 (28651352). According to the present invention, the above numbers into brackets are the PMID number of the articles referring to the circular RNAs.

Preferably, in the composition containing extracellular vesicles loaded with a molecule of interest for use in the treatment of osteoarthritis, the molecule of interest is at least one miRNA and at least one circular RNA chosen in the group of circular RNA consisting of hsa_circ_0110251 (30923232), hsa_circ_0015260 (31190410), hsa_circ_0005406 (31190410), hsa_circ_0005567 (28624198), hsa_circ_0092516 (33135071), hsa_circ_0020014 (31502887), hsa_circ_0094742 (31502887), hsa_circ_0000284/circHIPK3 (32767319), hsa_circ_0023404 (26931159), hsa_circ_0032131 (31526191), hsa_circ_0001979 (31190410), hsa_circ_0104873 (33675175), hsa_circ_0037658 (31190410), hsa_circ_0045714 (28795385), hsa_circ_0002485 (31111536), hsa_circ_0057421 (31502887), hsa_circ_0008172 (31190410), hsa_circ_0008365 (30923232), hsa_circ_0001103 (30923232), hsa_circ_0116061 (33849596), hsa_circ_0005105 (28276108), hsa_circ_0072568 (30923232), hsa_circ_0008667 (30923232), hsa_circ_0077425 (31190410), hsa_circ_0134111 (31502887), hsa_circ_0083429 (33520980), hsa_circ_0136474 (31402547), circRNA-9119 (32522568), hsa_circRNA_000598 (28624198), hsa_circRNA_100086 (26931159), hsa_circRNA_101178 (26931159), hsa circRNA_101914 (26931159), hsa circRNA_101975 (28624198), hsa_circRNA_103387 (28624198). According to the present invention, the above numbers into brackets are the PMID number of the articles referring to the circular RNAs.

Preferably, in the composition containing extracellular vesicles loaded with a molecule of interest for use in the treatment of rheumatoid arthritis, the molecule of interest is at least one miRNA and at least one circular RNA chosen in the group of circular RNA consisting of hsa_circ_0000367 (31772684), hsa_circ_0000396 (31596149), hsa_circ_0032683 (28983619), hsa_circ_0035197 (31772684), hsa_circRNA_101873 (27097056), hsa_circ_0008410 (32124964), hsa_circ_0052012 (28983619), hsa_circRNA_003524 (28618429), hsa_circRNA_103047 (28618429), hsa circ_103047 (28618429), hsa_circ_0001200 (32191279), hsa_circ_0002715 (31772684), hsa_circ_0008360 (32191279), hsa_circ_0064996 (28983619), hsa_circ_0130438 (31596149), hsa_circ_0083964 (28983619), hsa_circ_104871 (28618429), hsa_circ_0001859/hsa_circ_001783 (29577053), hsa_circ_0003972 (32191279), hsa_circ_0001947/hsa_circ_000510 (31772684). According to the present invention, the above numbers into brackets are the PMID number of the articles referring to the circular RNAs.

Preferably, in the composition containing extracellular vesicles loaded with a molecule of interest for use in the treatment of inflammation, the molecule of interest is at least one miRNA and at least one circular RNA chosen in the group of circular RNA consisting of hsa_circ_0035266 (33107911), hsa_circ_0000638 (32199215), hsa_circ_0004587 (31864925), hsa_circRNA_103516 (31749597), hsa_circ_0011785 (32113679), hsa_circ_0004831 (32113679), hsa_circ_0040824 (32113679), hsa_circ_0092316 (32113679), hsa_circ_0002194 (32113679). According to the present invention, the above numbers into brackets are the PMID number of the articles referring to the circular RNAs.

Preferably, in the composition containing extracellular vesicles loaded with a molecule of interest for use in the treatment of Arthrosis, the molecule of interest comprises (i) at least one miRNA chosen in the group of miRNAs consisting of miR-146a, miR-140, miR-126, miR-132, and (ii) at least an anti-mir-155. More preferably, the molecule of interest comprises at least one miRNA chosen in the group consisting of miR-146a, miR-140, miR-126, miR-132, and at least an anti-mir-155 in an amount equal or superior to 10%, more preferably 20%, 30%, 40%, 50%, 60% of all miRNA sequences present in the extracellular vesicles (copy number of at least one miRNA chosen in the group consisting of miR-146a, miR-140, miR-126, miR-132 and an anti-mir-155 present in the extracellular vesicles towards the total copy number of all miRNA sequences present in the extracellular vesicles). Even more preferably, in the composition containing extracellular vesicles loaded with a molecule of interest for use in the treatment of Arthrosis, the molecule of interest consists of a combination of (i) at least one miRNA chosen in the group of miRNAs consisting of miR-146a, miR-140, miR-126, miR-132, and (ii) an anti-miR-155.

Preferably, in the composition containing extracellular vesicles loaded with a molecule of interest for use in the treatment of NAFLD, the molecule of interest comprises at least miR-122 and at least an anti-mir-34a. More preferably, the molecule of interest comprises at least miR-122 and at least an anti-mir-34a in an amount equal or superior to 10%, more preferably 20%, 30%, 40%, 50%, 60% of all miRNA sequences present in the extracellular vesicles (copy number of miR-122 and anti-miR-34a present in the extracellular vesicles towards the total copy number of all miRNA sequences present in the extracellular vesicles). Advantageously, in the composition containing extracellular vesicles loaded with a molecule of interest for use in the treatment of NAFLD, the molecule of interest consists of a combination of miR-122 and an anti-mir-34a.

Preferably, in the composition containing extracellular vesicles loaded with a molecule of interest for use in the treatment of Psoriasis, the molecule of interest comprises at least miR-146a and at least an anti-mir-155. More preferably, the molecule of interest comprises at least miR-146a and at least an anti-mir-155 in an amount equal or superior to 10%, more preferably 20%, 30%, 40%, 50%, 60% of all miRNA sequences present in the extracellular vesicles (copy number of miR-146a and anti-miR-155 present in the extracellular vesicles towards to total copy number of all miRNA sequences present in the extracellular vesicles). Advantageously, in the composition containing extracellular vesicles loaded with a molecule of interest for use in the treatment of Psoriasis, the molecule of interest consists of a combination of miR-146a and an anti-mir-155.

Preferably, in the composition containing extracellular vesicles loaded with a molecule of interest for use in the treatment of diabetic foot ulcer, the molecule of interest comprises at least miR-128p and at least an anti-mir-198. More preferably, the molecule of interest comprises at least miR-128p and at least an anti-mir-198 in an amount equal or superior to 10%, more preferably 20%, 30%, 40%, 50%, 60% of all miRNA sequences present in the extracellular vesicles (copy number of miR-128p and anti-mir-198 present in the extracellular vesicles towards to total copy number of all miRNA sequences present in the extracellular vesicles). Advantageously, in the composition containing extracellular vesicles loaded with a molecule of interest for use in the treatment of diabetic foot ulcer, the molecule of interest consists of a combination of miR-128p and an anti-mir-198.

Preferably, in the composition containing extracellular vesicles loaded with a molecule of interest for use in the treatment of lung fibrosis, the molecule of interest comprises at least miR-29b and at least an anti-mir-199. More preferably, the molecule of interest comprises at least miR-29b and at least an anti-mir-199 in an amount equal or superior to 10%, more preferably 20%, 30%, 40%, 50%, 60% of all miRNA sequences present in the extracellular vesicles (copy number of miR-29b and anti-mir-199 present in the extracellular vesicles towards to total copy number of all miRNA sequences present in the extracellular vesicles). Advantageously, in the composition containing extracellular vesicles loaded with a molecule of interest for use in the treatment of fibrosis lung, the molecule of interest consists of a combination of miR-29b and an anti-mir-199.

According to the present invention, the RNA molecules, i.e. the circular RNA (circRNA) and miRNA molecules, indicated are the human circRNA and miRNA molecules, although the scope of the present invention is not limited to humans. The man skilled in the art would easily find the corresponding RNA molecules, i.e. the circular RNA (circRNA) and miRNA molecules, in other species.

It is understood that the present invention is in no way limited to the embodiments described above and that many modifications can be made thereto without departing from the scope of the appended claims.

## Claims

1. Method for the production of extracellular vesicles loaded with one or several molecule of interest compatible with the pharmaceutical industry, for example exosomes loaded with one or several molecule of interest comprising the following steps:
- a supply of a bioreactor chamber containing a porous three-dimensional scaffold by a cell culture medium via a supply port, and, after a predefined duration, an exit via an outlet port,
- seeding said porous three-dimensional scaffold with a starting number of cells in a volume of culture medium,
- a phase of attachment of said cells to form adherent cells on said porous three-dimensional scaffold,
- an expansion of the adherent cells on said porous three-dimensional scaffold up to a second number of adherent cells, said second number of adherent cells divided by said starting number defining a multiplication factor of at least 5 for a single expansion step in the said scaffold,
- a production of extracellular vesicles by said adherent cells during a production phase, subsequent to or concomitantly with the expansion, during which the vesicles are produced and excreted in the culture medium to give a culture medium enriched with extracellular vesicles,
- optionally, a first purification of the culture medium enriched with extracellular vesicles in order to produce a purified culture medium enriched with extracellular vesicles,- a loading of said extracellular vesicles with said one or several molecule of interest, subsequently to or concomitantly with the production,
- optionally, a (second) purification arranged to remove unloaded extracellular vesicles in order to produce a purified culture medium enriched with extracellular vesicles loaded with a molecule of interest,
- a harvest of extracellular vesicles.

2. Method according to claim 1, wherein said porous three-dimensional scaffold is a porous three-dimensional printed scaffold and/or having a specific surface area and a total volume in a ratio comprised between 25 m²/m³ and 250 m²/m³.

3. Method according to claim 1 or claim 2, wherein the adherent cells are adherent immortalized cells.

4. Method according to anyone of the preceding claims, wherein the molecule of interest is shRNA, miRNA, mRNA, gRNA, pri-miRNA, pre-miRNA, circular RNA, piRNA, tRNA, rRNA, snRNA, IncRNA, ribozymes, mini-circle DNA, plasmid DNA, circular RNA, siRNA, agomir, antagomir or a combination thereof, preferably circular RNA and miRNA.

5. Method according to anyone of the preceding claims, wherein said phase of loading said extracellular vesicles with said molecule of interest is performed through
(i) a transfection step of said adherent cells during or after said expansion with a vector encoding said molecules of interest and/or directly with said molecules of interest, and/or
(ii) a mechanoporation step after said harvest of extracellular vesicles, and/or
(iii) the intracellular incorporation of the molecule of interest stably expressed by the engineered adherent immortalized cells into the extracellular vesicles.

6. Method according to anyone of the preceding claims, wherein said adherent cells are cells forming part of a group of cells consisting of immortalized immune regulatory cells, immortalized mesenchymal stem cells, immortalized Wharton's Jelly derived-mesenchymal stem cells, immortalized bone marrow-derived mesenchymal stromal cells, immortalized hTERT-mesenchymal stem cells, and/or immortalized induced pluripotent stem cells or cells derived from induced pluripotent stem cells, preferably immortalized mesenchymal stem cells.

7. Pharmaceutical composition containing extracellular vesicles loaded with a molecule of interest obtained by the method according to any one of claims 1 to 6.

8. Pharmaceutical composition according to claim 7, wherein said extracellular vesicles are exosomes obtained from adherent immortalized cells, preferably from immortalized mesenchymal stem cells.

9. Pharmaceutical composition according to claim 7 or claim 8, wherein said molecule of interest comprises at least one circular RNA.

10. Pharmaceutical composition according to anyone of claims 7 to 9, wherein said molecule of interest comprises at least one miRNA.

11. Pharmaceutical composition according to claim 7 or claim 8, wherein said molecule of interest comprises
a combination of (i) miR-146a and miR-140, and/or (ii) hsa-circRNA_0001236, or
a combination of miR-146 and anti-miR-155, or
a combination of (i) miR-146 or miR-140, and (ii) a circular RNA, or
a combination of (i) miR-140 and miR-146, and (ii) a circular RNA, or
a combination of miR-140 and anti-miR-155.

12. Pharmaceutical composition comprising a therapeutically effective amount of extracellular vesicles loaded with a molecule of interest wherein said molecule of interest comprises at least a circular RNA, and wherein said extracellular vesicles are extracellular vesicles obtained from adherent immortalized mesenchymal stem cell.

13. Pharmaceutical composition comprising a therapeutically effective amount of extracellular vesicles loaded with a molecule of interest wherein said molecule of interest comprises at least a miRNA, and wherein said extracellular vesicles are extracellular vesicles obtained from adherent immortalized mesenchymal stem cell.

14. Pharmaceutical composition comprising a therapeutically effective amount of extracellular vesicles loaded with a molecule of interest wherein said molecule of interest comprises at least a circular RNA and at least a miRNA.

15. Pharmaceutical composition according to anyone of claims 7 to 14, for use in the treatment of a pathology selected from a group consisting of arthrosis, Alzheimer disease, liver disease, diabetic diseases, lung fibrosis, atherosclerosis, osteoarthritis, facet joint osteoarthritis, multiple sclerosis, inflammation and cancer.
